# EUROPÄISCHE PATENTSCHRIFT

(11) **EP 1 932 494 B1**
(45) Veröffentlichungstag und Bekanntmachung des Hinweises auf die Patenterteilung: **17.06.2009**
(21) Anmeldenummer: 06025672.4
(22) Anmeldetag: 12.12.2006
(51) Int. Cl.: A61F 2/46, A61F 2/30

(54) **Bestimmung einer Gelenkorientierung für eine Implantation**
Determination of a joint orientation for implantation
Determination de l'orientation d'un joint pour une implantation

(43) Veröffentlichungstag der Anmeldung: 18.06.2008
(73) Patentinhaber: BrainLAB AG, 85622 Feldkirchen (DE)
(72) Erfinder: Warkentine, Blaine, M.D., Long Beach CA 90803 (US); Dick, Robert, 80686 München (DE)
(74) Vertreter: Schwabe - Sandmair - Marx

(56) Entgegenhaltungen:
- EP-A1- 1 402 855
- WO-A-02/080824
- DE-A1- 19 709 960
- US-B1- 6 205 411

## Beschreibung

Die vorliegende Erfindung betrifft die Bestimmung einer Orientierung eines anatomischen Gelenks für die Planung der Implantation eines künstlichen Gelenks sowie eine Vorrichtung zum Bestimmen der Orientierung zur Implantation eines künstlichen Gelenks. Das künstliche Gelenk wird implantiert, um ein bereits im menschlichen oder tierischen Körper vorhandenes Gelenk zu ersetzen. Dieses zu ersetzende Gelenk wird im Folgenden anatomisches Gelenk genannt. Das anatomische Gelenk kann aus bestimmten Gründen, z.B. Unfall oder Krankheit (z.B. Osteoporose) defekt oder nur noch eingeschränkt funktionsfähig sein oder Schmerzen verursachen. Um Komplikationen nach der Implantation eines künstlichen Gelenks möglichst zu vermeiden, ist eine Implantation des künstlichen Gelenks mit einer geeigneten Orientierung von Vorteil. Das Dokument US-B-6 205 411 stellt der nächste Stand der Technik dar.

Aus EP 1 402 855 A1 ist eine Vorrichtung und ein Verfahren zum Bestimmen des Öffnungswinkels eines Gelenks bekannt. Die Vorrichtung wird verwendet, um den richtigen Sitz eines implantierten Gelenks zu überprüfen. Hierzu wird ein Öffnungswinkel des Gelenks bestimmt.

Aus DE 197 09 960 A1 ist ein Verfahren und eine Vorrichtung zur präoperativen Bestimmung der Positionsdaten von Endoprothesenteilen bestimmt. Die Orientierung einer Kniegelenk-Endoprothese soll relativ zu charakteristischen Richtungen bestimmt werden. Die charakteristischen Richtungen werden durch Verbindung eines hüftnahen Gelenkpunktes und eines knienahen Gelenkpunktes für den Oberschenkel bestimmt. Für den Unterschenkel wird die charakteristische Richtung durch die geradlinige Verbindung des fußnahen Gelenkpunktes mit dem knienahen Gelenkpunkt bestimmt.

Aufgabe der Erfindung ist es, ein Verfahren und eine Vorrichtung bereitzustellen, um die Planung einer Implantation eines künstlichen Gelenks mit einer geeigneten Orientierung und/oder an einer geeigneten Lage zu unterstützen.

Vorstehende Aufgabe wird durch die Gegenstände der unabhängigen Ansprüche gelöst. Vorteilhafte Weiterbildungen gehen aus den Unteransprüchen hervor.

Das künstliche Gelenk, das erfindungsgemäß ein anatomisches Gelenk, wie z.B. ein Hüftgelenk oder ein Knie-, Fuß-, Finger- oder Schultergelenk ersetzen soll, hat vorzugsweise mindestens zwei Teile, die relativ zueinander beweglich sind und eine Gelenkverbindung eingehen können. Ein Beispiel für einen (ersten) Teil eines künstlichen Gelenks ist eine Gelenkpfanne und ein Beispiel für den dazu gehörigen anderen (zweiten) Teil ist ein Gelenkkopf, der beispielsweise an einem Schaft angebracht ist. Kopf und Pfanne sind zueinander passend ausgebildet. Beispielsweise kann der erste Teil die Gelenkpfanne und der zweite Teil der Gelenkkopf sein. Die Benennung "erste" und "zweite" ist hierin willkürlich und kann auch anders herum gewählt werden. Sie bringt lediglich zum Ausdruck, dass es sich um zwei kooperierende Teile eines Gelenks handelt.

Der erste Teil des künstlichen Gelenks ist vorzugsweise ausgebildet, um in eine erste Körperstruktur (z.B. ein Knochen oder ein Knorpel oder eine Mischung daraus) implantiert werden. Beispielsweise kann eine künstliche Gelenkpfanne in einen Beckenknochen implantiert werden, wobei die künstliche Gelenkpfanne beispielsweise den ersten Teil darstellt und das Becken (Pelvis) die erste Körperstruktur. Weiter kann bei einer Totalendoprothese der künstliche Kugelgelenkkopf zusammen mit einem Schaft in einem Oberschenkelknochen (Femur) implantiert werden, wobei beispielsweise der Kugelgelenkkopf zusammen mit dem Schaft den zweiten Teil des künstlichen Gelenks darstellt und der Oberschenkelknochen die zweite Körperstruktur darstellt.

Erfindungsgemäß wird vorzugsweise aber nicht obligatorisch ein Implantat-Datensatz bereitgestellt. Dieser kann beispielsweise in einem Speicher abgespeichert sein oder über eine Eingabeschnittstelle (z.B. Netzwerkanschluss, Tastatur etc.) eingegeben werden. Der Implantatdatensatz umfasst vorzugsweise Daten, die eine Bewegbarkeit des ersten Teils relativ zum zweiten Teil betreffen.

Was der Begriff "Bewegbarkeit" umfasst, wird im Folgenden anhand des künstlichen Gelenks erläutert, trifft aber analog auch auf das anatomische Gelenk zu, wobei der erste Teil des künstlichen Gelenks der ersten Körperstruktur entspricht und der zweite Teil des künstlichen Gelenks der zweiten Körperstruktur. Der Begriff umfasst insbesondere einen Bewegungsbereich des künstlichen Gelenks, also beispielsweise die möglichen Winkel, die der erste Teil des künstlichen Gelenks relativ zum zweiten Teil einnehmen kann, insbesondere maximal mögliche Winkel. Diese Winkel können für Ebenen definiert sein, die beispielsweise senkrecht zueinander stehen und insbesondere charakteristische Ebenen (Schlüsselebenen) des künstlichen Gelenks darstellen. Insbesondere können sie maximale Bewegungsbereiche darstellen, die das künstliche Gelenk erlaubt. Zum Beispiel kann es im Rahmen einer Relativbewegung zu einem Anstoßen des zweiten Teils (z.B. Kugeln mit Schaft) an dem ersten Teil (z.B. Pfanne) kommen. Die Bewegungsbereiche können z.B. durch Winkel beschrieben werden, die für bestimmte Ebenen definiert werden, in denen insbesondere eine Bewegung des ersten Teils relativ zum zweiten Teil möglich ist. Weiter kann die Bewegbarkeit mögliche Lagen, insbesondere Extremlagen oder Schlüssellagen umfassen, bei denen der erste Teil relativ zum zweiten Teil eine bestimmte Lage, insbesondere Extremlage oder Schlüssellage einnimmt. Ein Beispiel für eine Extremlage ist gegeben, wenn der erste Teil relativ zum zweiten Teil den Rand eines möglichen Bewegungsbereichs erreicht hat und die beiden Teile aneinander anstoßen, so dass eine Bewegung nur noch in eine Richtung, weg von der Extremlage (Anstoßpunkt) und zurück zu einer bereits vorher eingenommenen Relativlage im Rahmen einer üblichen Gelenkbewegung möglich ist. Eine übliche Gelenkbewegung ist eine Bewegung, bei der die beiden Teile des Gelenks (erster und zweiter Teil) nicht getrennt werden sondern Bewegungen ausführen, wie sie insbesondere den (gesunden) Bewegungen des anatomischen Gelenks entsprechen. Die Lagen können z.B. in einem Bezugssystem beschrieben werden, das im ersten oder im zweiten Teil verankert ist und beispielsweise mit kartesischen Koordinaten oder Kugelkoordinaten oder Winkeln relativ zu den Ebenen beschrieben werden. Insbesondere können die relativen Lagen von Symmetrieachsen oder -ebenen im ersten Teil relativ zu Symmetrieachsen oder -ebenen im zweiten Teil beschrieben werden oder die Positionen bestimmter Elemente (z.B. Landmarken) des ersten Teils relativ zu Positionen bestimmter Elemente des zweiten Teils (beispielsweise der tiefste Punkt einer schalenförmigen Vertiefung eines Pfannengelenks) beschrieben werden.

Weiter kann der Implantat-Datensatz Daten über die Geometrie des anatomischen Gelenks umfassen. Daten über die Geometrie sind beispielsweise Daten über die Form und/oder Gestaltung des künstlichen Gelenks. Die Bewegbarkeit kann durch die Geometrie und/oder durch das Material oder die Materialien beeinflusst werden, die das künstliche Gelenk umfasst oder aus denen es gebildet ist, insbesondere die Elastizität, Flexibilität und/oder Starrheit der Materialien. Letztere Eigenschaften und/oder die Geometrie können insbesondere beeinflussen, mit welchen Kräften sich die Teile des künstlichen Gelenks (erster und zweiter Teil) voneinander trennen lassen. Die Bewegbarkeit umfasst zudem vorzugsweise Informationen über eine mögliche relative Bewegung des ersten Teils und zweiten Teils im Normalfall, bei welchem das künstliche Gelenk eine Bewegung des gesunden anatomischen Gelenks nachahmt. Vorzugsweise umfasst die Bewegbarkeit auch Information über eine Bewegung des ersten Teils relativ zu dem zweiten Teil, bei dem sich das erste Teil vom zweiten Teil trennt, wie dies insbesondere unerwünscht ist und wie dies bei einer Luxation der Fall sein kann. Diese unerwünschte relative Bewegung wird insbesondere auch durch die Geometrie des künstlichen Gelenks beeinflusst. Insbesondere beeinflusst die Geometrie den Grad des Widerstandes, den das künstliche Gelenk gegen eine unerwünschte Trennung des ersten und zweiten Teils entgegensetzt.

Der Implantat-Datensatz umfasst somit vorzugsweise auch Daten über die Hemmung einer Bewegung, die eine Trennung des ersten Teils und des zweiten Teils bewirkt, also insbesondere die Hemmung einer Luxationsbewegung. Insbesondere umfasst er Daten über den Grad der Hemmung einer derartigen Bewegung oder über den Widerstand, der einer solchen Bewegung entgegengesetzt wird. Der Grad der Hemmung und/oder der Widerstand, kann sich insbesondere aus der Geometrie des künstlichen Gelenks ergeben und kann insbesondere durch geometrische Eigenschaften ausgedrückt werden, wie weiter unten bei der detaillierten Beschreibung näher ausgeführt wird. Auch kann er durch Kräfte, beispielsweise Reibungskräfte oder Haltekräfte ausgedrückt werden.

Der Implantat-Datensatz kann beispielsweise einer Datenbank entnommen werden, die für die einzelnen Typen von Implantaten die entsprechenden Daten speichert. Auch können die Eigenschaften des Implantats vermessen werden, indem beispielsweise Relativbewegungen zwischen dem ersten und zweiten Teil ausgeführt werden und indem zum Beispiel bei einer Stellung (z.B. Schlüsselstellung) oder bei bestimmten relativen Stellungen des ersten und zweiten Teils versucht wird, den ersten Teil von dem zweiten Teil zu trennen und die entsprechenden Kräfte, die hierzu notwendig sind, gemessen werden. Diese Kräfte können dann als Daten dem Implantat-Datensatz hinzugefügt werden bzw. diesen bilden.

Erfindungsgemäß werden vorzugsweise Körperstruktur-Daten bereitgestellt, die insbesondere patientenspezifisch sind. Diese umfassen insbesondere Informationen über eine Bewegbarkeit des anatomischen Gelenks, also insbesondere der ersten und/oder zweiten Körperstruktur. Der Begriff der "Bewegbarkeit" wird hierbei analog zu dem oben erläuterten Begriff der Bewegbarkeit verstanden und umfasst insbesondere Informationen über mögliche relative Bewegungen und/oder relative Lagen der ersten Körperstruktur relativ zur zweiten Körperstruktur.

Vorzugsweise umfassen die Körperstruktur-Daten Informationen über die Lage der ersten Körperstruktur und/oder zweiten Körperstruktur, insbesondere über eine relative Orientierung der ersten Körperstruktur relativ zu der zweiten Körperstruktur. Die Lage und/oder Orientierung kann in einem vorbestimmten Bezugssystem beschrieben werden, z.B. in einem Bezugssystem des Patienten, in einem Bezugssystem eines verwendeten Navigationssystems (z.B. Bezugssystem in dem eine Detektionseinrichtung, z.B. Kamera des Navigationssystems ruht, in einem Bezugssystem, in dem der Operationsraum ruht usw. beschrieben werden) oder in einem Bezugssystem, das in einer der Körperstrukturen ruht. Umfassen beispielsweise die Informationen über die Lage sowohl Informationen über die Lage der ersten als auch über die Lage der zweiten Körperstruktur, so kann die Lage zumindest einer der beiden Körperstrukturen in einem Bezugssystem beschrieben werden, in dem die andere Körperstruktur ruht.

Die Körperstruktur-Daten, die die Bewegbarkeit der ersten relativ zur zweiten Körperstruktur betreffen, umfassen insbesondere mindestens einen Bewegungsbereich des anatomischen Gelenks, also insbesondere relative Bewegungen der ersten Körperstruktur relativ zur zweiten Körperstruktur. Insbesondere umfassen sie mindestens einen maximalen möglichen Bewegungsbereich, insbesondere zwei Bewegungsbereiche. Die Bewegungsbereiche werden bevorzugt für bestimmte Bewegungsrichtungen bzw. Bewegungsebenen beschrieben. Im Falle eines Hüftgelenkimplantats, erfolgt die Beschreibung beispielsweise in Richtung der Extension/Flexion des anatomischen Gelenks und/oder in der Richtung der Adduktion und Abduktion des Gelenks.. Insbesondere umfasst die Bewegbarkeit eine oder mehrere bestimmte Schlüssellagen der ersten Körperstruktur relativ zur zweiten Körperstruktur, also beispielsweise eine Angabe über eine Extremlage und/oder über einen maximalen Abduktionswinkel zwischen der ersten Körperstruktur (Gelenkpfanne) und der zweiten Körperstruktur (Gelenkkopf), um für diesen Fall das Risiko einer möglichen Luxation zu berechnen oder abzuschätzen, wie weiter unten aufgeführt wird.

Die Informationen über die Bewegbarkeit können insbesondere auch Daten über die Geometrie des anatomischen Gelenks umfassen, insbesondere Daten über die Gestalt und/oder Form des anatomischen Gelenks. Diese Daten können durch Diagnoseverfahren, wie z.B. Kernspinaufnahmen, Röntgenaufnahmen, Computertomographieaufnahmen (CT) usw. gewonnen werden. Insbesondere können Informationen über die Bewegbarkeit Daten über die Hemmung einer Bewegung und/oder den Widerstand gegen eine Bewegung der ersten und/oder zweiten Körperstruktur umfassen. Insbesondere können von dem Betriff der Bewegbarkeit Daten über Bänderspannungen, Lage von Bändern und Gewebestrukturen, die die Bewegung und den Bewegungsbereich des anatomischen Gelenks beeinflussen, umfasst sein. Insbesondere können typische (übliche) Werte in den Daten über die Bewegbarkeit enthalten sein, z.B. typische Werte über den Widerstand gegen eine Luxation und/oder typische Werte über Bewegungsbereiche, wie sie z.B. typischerweise (üblicherweise) bei einem Menschen gegeben sind.

Vorzugsweise wird erfindungsgemäß basierend auf den Körperstruktur-Daten eine Orientierung des anatomischen Gelenks bestimmt, die für die Planung der Implantation des künstlichen Gelenks verwendbar ist, und insbesondere die Bestimmung einer für die Implantation geeignete Orientierung des künstlichen Gelenks erlaubt. Erfindungsgemäß kann also eine Bestimmung der Orientierung des anatomischen Gelenks erfolgen. Diese bestimmte Orientierung kann dann von einem Planungsverfahren oder einem Arzt verwendet werden, um eine Implantation zu planen, insbesondere eine geeignete Lage und/oder Orientierung des künstlichen Gelenks zu bestimmen. Auch kann erfindungsgemäß eine geeignete Orientierung des künstlichen Gelenks direkt aus den Körperstrukturdaten bestimmt werden, indem beispielsweise Bewegungsbereiche des anatomischen und künstlichen Gelenks abgeglichen werden, ohne dass die Orientierung des anatomischen Gelenks bestimmt wird. Die Bestimmung der Orientierung des anatomischen Gelenks kann aber auch ein Zwischenschritt zur Bestimmung der geeigneten Orientierung des künstlichen Gelenks sein.

Die bestimmte Orientierung des anatomischen Gelenks kann dann als Anhaltspunkt für eine zur Implantation geeigneten Orientierung des künstlichen Gelenks verwendet werden. Insbesondere kann die Implantation so geplant werden, dass die Orientierung des künstlichen Gelenks mit der bestimmten Orientierung des anatomischen Gelenks übereinstimmt. Eine Übereinstimmung ist jedoch nicht obligatorisch, insbesondere wenn aus medizinischen Gründen für das künstliche Gelenk eine vom anatomischen Gelenk abweichende Orientierung gewünscht wird.

Vorzugsweise erfolgt die Bestimmung der geeigneten Orientierung des künstlichen Gelenks auch basierend auf dem mindestens einen Implantat-Datensatz. Dabei ist die bestimmte Orientierung des künstlichen Gelenksgeeignet für eine Implantation des ersten und/oder zweiten Teils des künstlichen Gelenks in einer Körperstruktur. Insbesondere ist diese Orientierung geeignet für eine Implantation des ersten Teils des künstlichen Gelenks (z.B. künstliche Gelenkpfanne) in eine erste Körperstruktur (z.B. Beckenknochen) und/oder für eine Implantation eines zweiten Teils des künstlichen Gelenks (z.B. Kugel mit Schaft) in eine zweite Körperstruktur (z.B. Oberschenkelknochen).

Die geeignete Orientierung kann z.B. als Gerade oder als Vektor in verschiedenen Bezugssystemen beschrieben werden, wie sie schon oben in Zusammenhang mit der Lage der ersten und zweiten Körperstruktur diskutiert wurden. Insbesondere kann die geeignete Orientierung in einem Bezugssystem beschrieben werden, in dem der Patient ruht, oder in dem der Operationsraum ruht, oder in dem ein Navigationssystem bzw. dessen Detektionssystem ruht. Insbesondere kann die geeignete Orientierung des künstlichen Gelenks auch in einem Bezugssystem beschrieben werden, in dem die erste Körperstruktur oder die zweite Körperstruktur ruht oder in dem einer der Teile des künstlichen Gelenks ruht. Insbesondere kann die geeignete Orientierung unter Bezugnahme auf Symmetrieebenen oder Landmarken der ersten und/oder zweiten Körperstruktur angegeben werden, wie beispielsweise die Symmetrieebenen des Beckenknochens.

Die Orientierung eines Gelenks wird vorzugsweise über dessen Bewegbarkeit, insbesondere Bewegungsbereich und/oder dessen Symmetrieeigenschaften (z.B. Symmetrieachsen, Symmetriepunkte, Symmetrieebenen, insbesondere Längsachsen eines Gelenkteils) bestimmt.

Beispielsweise kann die Orientierung durch einen Vektor beschrieben werden, der seinen Ursprung in der Mitte des Grundes der schalenförmigen Vertiefung des Acetabulums hat. Alternativ kann er beispielsweise seinen Ursprung auch dort haben, wo sich der Drehpunkt des anatomischen Gelenks befindet.

Die Orientierung eines Gelenks, insbesondere des anatomischen Gelenks und/oder des künstlichen Gelenks kann beispielsweise wie folgt bestimmt werden. Ein Teil eines zweiteiligen Gelenks wird festgehalten, während der andere Teil den Rand des zulässigen Bewegungsbereichs des Gelenks abfährt. Dieser in sich geschlossenen Randlinie des Bewegungsbereichs kann dann ein Mittelpunkt zugeordnet werden. Alternativ kann ein Teil des Gelenks entlang zweier, insbesondere orthogonal zueinander liegender Hauptachsen bewegt werden und der Schnittpunkt dieser Hauptachsen wird als Mittelpunkt des Bewegungsbereichs definiert. Der vorgenannte Mittelpunkt des Bewegungsbereichs spannt nun zusammen mit dem Gelenkmittelpunkt (der insbesondere bei einer Relativbewegung der Gelenkteile ortsfest bleibt) eine Gerade auf, die die Orientierung des Gelenks darstellt.

Ist die Lage des Mittelpunkts des Drehgelenks nicht bekannt, so kann die Orientierung beispielsweise so festgelegt werden, dass der Rand des Bewegungsbereichs eine Ebene aufspannt und eine Mittelachse des Bewegungsbereichs normal zu dieser Ebene ist und durch den Mittelpunkt des durch den Rand umschriebenen Bereichs hindurch läuft. Auch können die Gelenke entlang jeweils einer Hauptbewegungsrichtung bewegt werden, wobei diese Richtungen beispielsweise senkrecht zueinander stehen können. Die durch die Bewegung aufgespannten Ebenen schneiden sich und die Schnittgerade kann als die Orientierung des Gelenks definiert werden.

Wie oben ausgeführt, kann aus dem Bewegungsbereich und insbesondere den relativen Lagen zwischen der ersten und zweiten Körperstruktur ein Mittelpunkt des Gelenks bestimmt werden (der bei den Relativbewegungen ortsfest ist). Dieser Mittelpunkt kann zur Bestimmung einer geeigneten Lage zur Implantation des künstlichen Gelenks herangezogen werden. Es kann also bestimmt werden, welche Lage der Drehpunkt des künstlichen Gelenks einnehmen sollte. Insbesondere kann diese Lage so gewählt werden, dass sie mit dem Drehgelenkmittelpunkt des anatomischen Gelenks übereinstimmt. Die geeignete Lage kann also durch die Position des Drehgelenkmittelpunkts im Raum, also in einem der vorgenannten Bezugssysteme bestimmt werden.
Vorzugsweise wird die geeignete Orientierung unter bestimmten Randbedingungen bestimmt, wie z.B. dass der Bewegungsbereich des künstlichen Gelenks den Bewegungsbereich des anatomischen Gelenks umfasst oder zumindest in etwa entspricht. "zumindest in etwa" bedeutet, dass falls der Bewegungsbereich durch Winkel beschrieben wird, eine Abweichung der beiden Bewegungsbereichte voneinander kleiner 20°, insbesondere kleiner 10°, 5°, oder 2° ist. Auf diese Art und Weise, ist gewährleistet, dass durch Bewegungen der ersten Körperstruktur relativ zur zweiten Körperstruktur kein Anschlag an die Grenzen des möglichen Bewegungsbereiches des künstlichen Gelenks erzielt werden kann. Ein derartiger Anschlag kann Komplikationen hervorrufen. Vorzugsweise erfolgt die Platzierung des künstlichen Gelenks dergestalt, dass die Orientierung des künstlichen Gelenks, also insbesondere die Mittelachse eines möglichen Bewegungsbereichs des künstlichen Gelenks (z.B. in einer bestimmten Ebene der Bewegung) mit der Orientierung, also insbesondere der Mittelachse des Bewegungsbereichs des anatomischen Gelenks (in einer entsprechenden Ebene) zumindest in etwa übereinstimmt. Dabei soll "zumindest in etwa" bedeuten, dass eine Abweichung der Mittelachsen voneinander kleiner als 20%, insbesondere kleiner als 10%, insbesondere kleiner als 5%, insbesondere kleiner als 2% des möglichen Bewegungsbereiches ist. Für das letzte genannte Beispiel von 2% würde dies bei einem maximalen Bewegungsbereich des künstlichen Gelenks von 100° bedeuten, dass die Abweichung der Mittelachse des Bewegungsbereichs des künstlichen Gelenks von der Mittelachse des Bewegungsbereichs des anatomischen Gelenks kleiner als +/- 2° ist. Die Mittelachse geht durch den Mittelpunkt des Gelenks sowie den Mittelpunkt des Bewegungsbereichs.

Alternativ oder zusätzlich zu der oben genannten Randbedingung der Bestimmung der geeigneten Orientierung anhand der Bewegungsbereiche des anatomischen und künstlichen Gelenks, kann die Bestimmung der geeigneten Orientierung wie im Folgenden dargestellt erfolgen. Dabei wird basierend auf dem Implantat-Datensatz, die Informationen über den Widerstand oder des Risikos einer Luxation enthalten, die geeignete Lage bestimmt. Insbesondere sind Informationen über eine Trennung des ersten Teils von dem zweiten Teil insbesondere für bestimmte relative Lagen des ersten Teils relativ zum zweiten Teil enthalten. Vorzugsweise wird bei der Bestimmung der geeigneten Orientierung ein Wert für einen Widerstand gegen eine unerwünschte Trennung berücksichtigt. Dieser vorbestimmte Wert eines Widerstandes oder gewünschten Widerstandes kann sich insbesondere an dem typischen Widerstand eines anatomischen Gelenks gegen eine Luxation in einer vorbestimmten relativen Lage der ersten Körperstruktur relativ zur zweiten Körperstruktur orientieren. Insbesondere kann der Widerstandwert einer anatomischen Datenbank entnommen werden. Insbesondere kann er so gewählt werden, dass er mit einem typischen anatomischen Wert für einen Widerstand übereinstimmt oder zumindest in etwa übereinstimmt, wobei der Begriff "zumindest in etwa" und die damit verbundenen Abweichungen vom dem typischen Wert im oben definierten Sinne verstanden werden kann und beispielsweise kleiner als 20 % sind. Ein anatomischer Wert für einen Widerstand kann experimentell bestimmt werden und kann so aufgefasst werden, dass er einem Gegendruck entspricht, den eine intakte Kapsel und die Bänder des Acetabulum einer Luxation entgegensetzen.

Vorzugsweise werden die anatomischen Daten, insbesondere die Daten über die relativen Lagen der ersten und zweiten Körperstruktur und/oder mögliche Bewegungsbereiche mit anatomischen Daten aus einer Datenbank verglichen, die übliche (typische) oder maximal mögliche Bewegungsbereiche und/oder relativen Lagen beschreiben, wie sie beim Menschen oder bei einem bestimmten Tier (üblicherweise) gegeben sind. Auf diese Art und Weise kann eine Plausibilitätsprüfung der eingegebenen anatomischen Daten durchgeführt werden. Insbesondere kann ein Warnsignal ausgegeben werden, falls die Überprüfung ergibt, dass die Daten von den üblichen Daten abweichen bzw. nicht innerhalb eines üblichen Wertebereichs liegen.

Vorzugsweise wird mindestens eine geeignete Orientierung bestimmt. Es kann also auch mehr als eine geeignete Orientierung bestimmt werden. Insbesondere umfasst der Begriff "mindestens eine Orientierung" auch dass Bereiche für eine geeignete Orientierung bestimmt werden. Ein Beispiel für Bereiche sind beispielsweise Winkelbereiche (beispielsweise bezogen auf Symmetrieebenen) in denen eine Implantation eines künstlichen Gelenks als geeignet angesehen wird. Beispielsweise können mehrere Winkel oder ein Winkelbereich für die Implantation eines künstlichen Gelenks dergestalt geeignet sein, dass der maximal mögliche Bewegungs-Winkelbereich des anatomischen Gelenks innerhalb des maximalen möglichen Bewegungs-Winkelbereichs des künstlichen Gelenks liegt. Derartige mehrere Orientierungen oder Orientierungsbereiche können insbesondere mittels einer Anzeigeeinrichtung (z.B. Monitor) oder allgemein einer Benutzerschnittstelle ausgegeben werden, so dass ein Bediener, insbesondere Operateur eine Orientierung aus den vorgeschlagenen Lagen auswählen kann oder eine Orientierung innerhalb des vorgeschlagenen Orientierungsbereichs auswählen kann. Die mehreren Orientierungen oder Orientierungsbereiche können auch basierend auf weiteren Nebenbedingungen, wie z.B. Minimierung des Risikos einer Luxation (siehe oben) eingeschränkt werden bzw. auf eine geeignete Lage reduziert werden.

Gemäß einer Ausführungsform der Erfindung können mehrere Implantat-Datensätze bereitgestellt werden, wobei jeder Implantat-Datensatz ein anderes künstlichen Gelenk betrifft. Die verschiedenen künstlichen Gelenke können insbesondere unterschiedliche Bewegbarkeiten aufweisen. Basierend auf den anatomischen Daten kann dann ein geeignetes künstliches Gelenk (mit dem diesen entsprechenden Implantat-Datensatz) oder mehrere geeignete künstliche Gelenke (mit den diesen entsprechenden Implantat-Datensätzen) ausgewählt werden.

Vorzugsweise wird ein künstliches Gelenk ausgewählt werden, für das der maximal mögliche Bewegungsbereich größer ist als der maximal mögliche Bewegungsbereich des zu ersetzenden anatomischen Gelenks. Die Auswahl kann automatisch erfolgen oder einem Benutzer können beispielsweise Vorschläge unterbreitet werden, so dass dieser dann die endgültige Auswahl trifft.

Das vorgehend beschriebene Verfahren zum Bestimmen einer Orientierung kann insbesondere bei einem erfindungsgemäßen Planungsverfahren zum Planen einer Implantation eines künstlichen Gelenks eingesetzt werden. Ein Bediener, beispielsweise ein Chirurg kann basierend auf dem Implantat-Datensatz und den Körperstruktur-Daten eine für eine Implantation geeignete Orientierung gemäß dem erfindungsgemäßen Verfahren bestimmen und somit basierend auf der geeigneten Orientierung die Implantation des künstlichen Gelenks planen. Die Bestimmung erfolgt dabei vorzugsweise so, dass nach Implantation des künstlichen Gelenks mit der geeigneten Orientierung das implantierte Gelenk alle relativen Lagen des ersten Teils relativ zum zweiten Teil einnehmen kann, die auch die erste und zweite Körperstruktur aufgrund des anatomischen Gelenks einnehmen konnten.

Die vorgenannten Verfahren können als ein Programm implementiert werden, das insbesondere auf einem computerlesbaren Speichermedium (z.B. CD oder DVD) gespeichert ist oder über eine Signalwelle (z.B. Internet-Downloadübertragung) übertragbar ist.

Weiter betrifft die vorliegende Erfindung eine Vorrichtung zum Bestimmen Orientierung eines anatomischen Gelenks und/oder einer für eine Implantation geeigneten Orientierung des künstlichen Gelenks, die insbesondere einen Speicher insbesondere zum Speichern des mindestens einen Implantat-Datensatzes und zum Speichern der Körperstruktur-Daten umfasst. Weiter umfasst sie eine Datenverarbeitungseinrichtung, die ausgebildet ist, um Schritte des vorgenannten Verfahrens durchzuführen, insbesondere um eine Bestimmung, insbesondere eine Berechnung der Orientierung des anatomischen Gelenks und/oder der geeigneten Orientierung des künstlichen Gelenks durchzuführen, wobei hierbei insbesondere numerische Verfahren und/oder analytische Verfahren eingesetzt werden können, um mathematisch die Orientierung (des anatomischen und/oder künstlichen Gelenks) basierend auf den Körperstruktur-Daten und vorzugsweise auch auf dem mindestens einen Implantat-Datensatz zu bestimmen. Sind mehrere Implantat-Datensätze vorhanden, wird vorzugsweise zuerst eine Einschränkung der Implantat-Datensätze beispielsweise auf einen Implantat-Datensatz vorgenommen, wie dies bereits oben beschrieben wurde. In einem nächsten Berechnungsvorgang (oder nächsten Schritt) wird dann die Orientierung (des anatomischen und/oder künstlichen Gelenks) bestimmt, wobei der ausgewählte Implantat-Datensatz, der dem ausgewählten künstlichen Gelenk entspricht, verwendet wird.

Vorzugsweise umfasst die Vorrichtung auch eine Detektionseinrichtung, um die Körperstruktur-Daten zumindest teilweise zu bestimmen. Die Detektionseinrichtung ist dabei vorzugsweise so ausgebildet, dass sie Markereinrichtungen detektieren kann, die an die erste und/oder zweite Körperstruktur angebracht sind. Auf diese Art und Weise kann bei Bewegung der ersten Körperstruktur relativ zur zweiten Körperstruktur (bzw. umgekehrt) der Bewegungsbereich und/oder mögliche Lagen der ersten Körperstruktur relativ zur zweiten Körperstruktur bestimmt werden. Diese so erhaltenen Daten können dann als Körperstruktur-Daten bei der Bestimmung der Orientierung (des anatomischen und/oder künstlichen Gelenks) verwendet werden.

Vorzugsweise ist die Vorrichtung so ausgebildet, dass sie eine Benutzerschnittstelle, insbesondere eine Hinweiseinrichtung (z.B. Bildschirm) umfasst, die ausgebildet ist, dem Benutzer bei der Bewegung zumindest einer von den beiden Körperstrukturen (erste und zweite Körperstruktur) Hinweise zu geben. Insbesondere ist die Hinweiseinrichtung so ausgebildet, dass sie den Benutzer auf noch fehlende Bewegungen der Körperstrukturen hinweist und/oder dem Benutzer Hinweise gibt, in welche Richtungen die Bewegungen zu erfolgen haben. Ist beispielsweise bisher nur eine Bewegung in einer Extension/Flexion-Richtung erfolgt, so kann die Hinweiseinrichtung (z.B. Bildschirm) den Bediener darauf hinweisen, dass eine Bewegung in einer Abduktion/Adduktion-Richtung noch fehlt, um ausreichend Körperstrukturdaten zur Bestimmung einer Orientierung zu haben. Insbesondere kann der Bediener aufgefordert werden, einzugeben, wenn eine relative Lage zwischen einer ersten Körperstruktur und einer zweiten Körperstruktur eine Extremlage oder Schlüssellage darstellt, die z.B. dem Rand eines möglichen Bewegungsbereiches der ersten Körperstruktur relativ zur zweiten Körperstruktur entspricht.

Bei der folgenden detaillierten Beschreibung werden weitere Merkmale der vorliegenden Erfindung offenbart. Merkmale verschiedener Ausführungsformen können miteinander kombiniert werden.
Figur 1 erläutert den Bewegungsbereich eines künstlichen Gelenks;
Figur 2 erläutert einen Luxationswiderstand;
Figur 3 zeigt schematisch den Aufbau einer erfindungsgemäßen Vorrichtung.

Bei der folgenden detaillierten Beschreibung wird beispielhaft als anatomisches Gelenk ein Hüftgelenk beschrieben. Insbesondere wird dabei der Bewegungsbereich des Hüftgelenks, genauer der gegebene Bewegungsbereich des (anatomischen) Hüftgelenks eines Patienten zur Bestimmung einer geeigneten Orientierung des künstlichen Gelenks verwendet.

Während eines chirurgischen Eingriffes für eine Totalendoprothese eines Hüftgelenks werden beide Teile der anatomischen Hüfte, die das anatomische Gelenk bewegen, durch zwei Teile eines künstlichen Gelenks (durch ein zweiteiliges Implantat) ersetzt. Das Acetabulum (Gelenkpfanne) innerhalb des Beckenknochens (Pelvis) wird durch ein Gelenkpfannenimplantat ersetzt. Der proximale Teil des Oberschenkelknochens wird durch eine im folgenden Schaftimplantat genannte Implantatskombination aus Schaft und Gelenkkopf ersetzt. Der Schaft wird hierbei meist in den proximalen Oberschenkelknochen eingesetzt während der Gelenkkopf meist auf den Schaft aufgesetzt wird.
Der Erfolg des chirurgischen Eingriffs hängt von der Stabilität des implantierten künstlichen Gelenks im Knochen (Becken bzw. Oberschenkelknochen) ab und hängt insbesondere auch von dem Bewegungsbereich ab, der von dem neuem künstlichen Gelenk ermöglicht wird. Der Bewegungsbereich kann die Stabilität beeinflussen. Ein zu geringer Bewegungsbereich verursacht das Anstoßen eines Teils des künstlichen Gelenks an dem andere Teil des künstlichen Gelenks und kann folglich dazu führen, dass die Stabilität des Implantats, d.h. die Verbindung des Implantats mit den Knochen leidet. Dies kann dazu führen, dass sich das Implantat lockert oder vollständig vom Knochen löst. Die damit für den Patienten verbundenen Komplikationen sind insbesondere ein begrenzter Bewegungsbereich, Schmerzen, das Risiko einer Dislokation und schließlich das Risiko frühzeitig sich einer erneuten Operation unterziehen zu müssen.

Die vorliegende Erfindung hilft dem Chirurgen dabei, eine möglichst gute Orientierung des künstlichen Gelenks, insbesondere der künstlichen Gelenkpfanne und/oder des künstlichen Gelenkkopfes zu finden, um somit einen möglichst guten Bewegungsbereich zu erzielen. Vorzugsweise wird zum Bestimmen der möglichst optimalen Orientierung und insbesondere zum Platzieren des künstlichen Gelenks der Bewegungsbereich des anatomischen Gelenks zugrunde gelegt. Dieser Bewegungsbereich des anatomischen Gelenks kann als Datensatz bereitgestellt werden, kann vor dem chirurgischen Eingriff bestimmt werden oder kann während des chirurgischen Eingriffes bestimmt werden. Basierend auf den Daten über den Bewegungsbereich (ROM für "range of motion") wird dann die Lage der künstlichen Gelenkpfanne und/oder des künstlichen Schaftimplantats, also von zwei Teilen des Implantats bestimmt, insbesondere die Platzierung mit der geeigneten Orientierung geplant und erfolgt insbesondere die Platzierung während des chirurgischen Eingriffes mit der als geeignet bestimmten Orientierung.

Gemäß der Erfindung ist es nicht erforderlich, sich bei der Bestimmung der geeigneten Orientierung an Ebenen des Beckens zu orientieren, obwohl dies natürlich ebenfalls durchgeführt werden kann. Orientiert man sich an den Ebenen eines Beckens, so werden zwei Winkel definiert, die sich auf die Ebenen des Beckens beziehen, nämlich die Inklination und die Anteversion. In der Literatur wird eine so genannte sichere Zone zur Implantation einer künstlichen Gelenkpfanne definiert. Diese sichere Zone beträgt beispielsweise 40° bis 60° für die Inklination und 10° bis 30° für die Anteversion. Die Zonen sind so gewählt, dass die Wahrscheinlichkeit einer Fehlorientierung minimiert ist. Die angegebenen Werte sind jedoch nur statistische Werte und sind nicht an die gegebene Anatomie des individuellen Patienten angepasst. Dies kann dazu führen, dass eine Implantation, die sich an den Werten der sicheren Zone orientiert, für einen individuellen Patienten nicht optimal sein kann. Weiter ist es erforderlich, die Ebenen, insbesondere Symmetrieebenen des Beckens zu bestimmen. Dies kann schwierig sein, falls sich der Patient in lateraler Position befindet, da es erforderlich ist, im Anterior-Superior-Bereich der kontra lateralen Iliak-Spina einen Punkt (ASIS-Punkt) detektieren zu müssen. Berücksichtigt man erfindungsgemäß den individuellen Bewegungsbereich des anatomischen Gelenks (eines bestimmten Patienten), so können ungewünschte und insbesondere schmerzhafte Spannungen des Ligaments, insbesondere der Bänder vermieden oder vermindert werden. Insbesondere erlaubt die vorzugsweise Berücksichtigung des individuellen anatomischen Bewegungsbereichs des anatomischen Gelenks die Berücksichtigung von relativen Extremlagen der beiden Teile des anatomischen Gelenks, insbesondere die Berücksichtigung maximaler Bewegungsbereiche von Schlüsselbewegungen. Vorzugsweise wird weiter sowohl eine geeignete Orientierung zum Platzieren der künstlichen Gelenkpfanne als auch zum Platzieren des künstlichen Schaftimplantats bestimmt und insbesondere bei der Planung des chirurgischen Eingriffes berücksichtigt. Da die Orientierung des Schaftimplantats und der Gelenkpfanne miteinander in Beziehung stehen, kann durch die Bestimmung der Orientierung für beide Teile des künstlichen Gelenks eine suboptimale Platzierung eines Teils, insbesondere des Schaftimplantats verhindert werden, insbesondere kann ein beschränkter Bewegungsbereich vermieden werden. Gemäß einer Ausführungsform der Erfindung wird der Bewegungsbereich des Hüftgelenks intraoperativ bestimmt, um die gewonnenen Daten dann beim Planen einer Orientierung zum Implantieren der Gelenkpfanne und/oder des Schaftimplantats zu nutzen. Um die geeignete Orientierung zur Implantation des künstlichen Gelenks zu bestimmen, werden insbesondere die folgenden beiden Analysekonzepte eingesetzt, die auch miteinander kombiniert werden können.

Gemäß einem Konzept werden die maximalen Bewegungsbereiche von Schlüsselbewegungen bestimmt. Dabei kann es sich beispielsweise um Flexions-/Extensionsbewegungen, eine interne/externe Rotation oder eine Adduktion-/Abduktion-Bewegung oder andere kombinierte Bewegungen, wie z.B. eine interne Rotation während einer Flexionsbewegung handeln. Gemäß einem anderen Konzept stützt man sich auf eine für eine Dislokation (Luxation) kritische Richtung der Bewegung. Analysiert man den Bewegungsbereich eines anatomischen Gelenks, so werden vorzugsweise Daten gewonnen, die zumindest bei einem der vorgenannten Konzepte genutzt werden können, so dass die gewonnenen Daten verwendet werden können, um eine geeignete Lage für eine Gelenkpfanne und/oder ein Schaftimplantat zu bestimmen, um hierauf basierend eine Implantation des künstlichen Gelenks zu planen. Ein Operateur kann dann die Gelenkpfanne und/oder das Schaftimplantat basierend auf der Planung an der bestimmten geeigneten Position platzieren, indem insbesondere ein Navigationssystem verwendet wird, bei dem beispielsweise Markereinrichtungen an den Teilen des künstlichen Gelenks und/oder an den Körperstrukturen, in die die Teile des künstlichen Gelenks zu implantieren sind, angebracht sind. Die Markereinrichtungen werden vorzugsweise von dem Navigationssystem detektiert, um die Lage der Teile des künstlichen Gelenks zu bestimmen, insbesondere relativ zu den vorgenannten Körperstrukturen zu bestimmen, um so den Operateur während der Operation wertvolle Informationen über die Lage, insbesondere relative Lage der beteiligten Körperstrukturen und des künstlichen Gelenks zu liefern.

Ein Registrierungsverfahren, das z.B. mithilfe einer Detektionseinrichtung, die Markereinrichtungen detektiert und insbesondere mithilfe eines Navigationssystems durchgeführt werden kann, umfasst z.B. mindestens einen der folgenden Registrierungsschritte:
a) Eine Markereinrichtung (z.B. Referenzstern) wird zum Erfassen der Lage und insbesondere zum Verfolgen der Bewegung der Lage des Beckens und/oder des Oberschenkelknochens an dem Becken und/oder dem Oberschenkelknochen angebracht.
b) Die Lage eines ASIS-Punktes wird z.B. mithilfe eines Pointers erfasst. Alternativ oder zusätzlich kann z.B. mittels eines Pointers ein Punkt auf dem Schambein erfasst werden. Ein Pointer ist ein Zeigegerät, an dem mindestens zwei Markerkugeln angebracht sind und für den die relative Lage zwischen der Spitze des Pointers und der Markerkugeln bekannt ist, so dass mit der Spitze Landmarken und insbesondere Punkte abgegriffen und deren Lage durch Detektion der Markerkugeln mittels einer Detektionseinrichtung, insbesondere durch ein Navigationssystem erfasst und somit registriert werden können.
c) Das Bein des Patienten wird in eine neutrale Lage gebracht. Diese Position des Oberschenkelknochens gegenüber dem Becken wird als neutrale Lage abgespeichert.
d) Landmarken eines Oberschenkels und/oder den Oberschenkel kennzeichnende geometrische Eigenschaften, wie z.B. Symmetrieebenen oder Symmetrieachsen, beispielsweise die Achse des Schaftes des Oberschenkelknochens oder die Achse des Oberschenkelhalses werden beispielsweise mit Markereinrichtungen, Pointern und/oder durch Diagnoseeinrichtungen (wie z.B. CT oder Röntgenaufnahmen) bestimmt.
e) Der Bewegungsbereich des Oberschenkelknochens, insbesondere relativ zu dem Becken und/oder in einem vorbestimmten Bezugssystem (für das bereits oben Beispiele gegeben wurden) wird bestimmt. Die Bestimmung erfolgt insbesondere für eine vorbestimmte anatomische Situationen, wie z.B. die maximale Flexion, die maximale Extension, die maximale Abduktion, die maximale Adduktion und für kombinierte Bewegungen.
f) Oberflächenpunkte des Acetabulum (Hüftgelenkpfanne) werden erfasst.

Das unter b) Aufgeführte wird insbesondere verwendet, um ein skaliertes Beckenmodell bereitzustellen. Das oben unter c) Aufgeführte wird insbesondere eingesetzt, um eine Ausgangssituation oder Null-Situation zu definieren. Diese Ausgangssituation kann dann verwendet werden, um als Bezugspunkt für Schlüsselbewegungen (wie z.B. Flexionsbewegung, Extensionsbewegung, Adduktionsbewegung oder Abduktionsbewegung) zu dienen. Insbesondere kann die Ausgangssituation als Bezugspunkt für die Ränder des maximalen Bewegungsbereichs dienen. Die Ausgangssituation wird ebenfalls vorzugsweise gespeichert und dient insbesondere zur Bestimmung der Beinlänge und zur Berechnung eines Versatzes (Offset).

Der obige Punkt e) kann durch folgendes optionale Merkmal noch ergänzt werden. Die Erfassung des Bewegungsbereichs kann durch Plausibilitätsüberprüfungen abgesichert werden. Ein Beispiel hierfür stellt ein kleinster akzeptabler bzw. üblicher Flexionsbereich dar, wie er beim Menschen üblicherweise gegeben ist. Z.B. kann der kleinste noch akzeptable Flexionsbereich 110° betragen. Ein Bediener kann durch Warnhinweise darauf hingewiesen werden, falls die vorgenannten Plausilibitätskriterien nicht erfüllt sind, also falls beispielsweise ein Flexionsbereich kleiner 110° festgestellt wird.

Das erfindungsgemäße Verfahren und die erfindungsgemäße Vorrichtung, insbesondere ein Programm, das, wenn es auf einem Computer ausgeführt wird oder in einem Computer geladen wird, das erfindungsgemäße Verfahren durchführt, kann so gestaltet sein, dass es den Bediener unterstützt, wenn dieser die anatomischen Daten erfasst oder ergänzt. Insbesondere kann der Bediener (z.B. Chirurg) dabei von dem Programm unterstützt werden, wenn er noch nicht den Bewegungsbereich des anatomischen Gelenks voll bzw. ausreichend erfasst hat. Insbesondere kann basierend auf statistischen Modellen das Programm den Bediener unterstützen, unvollständige Körperstrukturdaten zu vervollständigen, so dass insbesondere die möglichen Bewegungen, bevorzugt Bereiche von Schlüsselbewegungen erfasst sind.

Vorzugsweise wird demnach basierend auf zumindest einem der folgenden beiden Analysekonzepte eine geeignete Orientierung für das künstliche Gelenk oder einem Teil davon, z.B. für die Gelenkpfanne berechnet:
a) Maximaler Bereich von Schlüsselbewegungen.
b) Kritische Richtung für eine Dislokation (Luxation)

Insbesondere sind die beiden obigen Konzepte auf eine Platzierung mit geeigneter Orientierung der Gelenkpfanne in einem ersten Schritt gerichtet. Da jedoch die Implantation der Gelenkpfanne und die Implantation des Schaftimplantats miteinander verbunden sind, umfasst vorzugsweise eine vollständige Planung ebenso die Bestimmung einer geeigneten Orientierung des Schaftimplantats. Während die Orientierung der Gelenkpfanne z.B. relativ zur Mediansagittalebene der Patientien angegeben werden kann, wird die Orientierung des Schaftimplantats (insbesondere des Schaftteils des Schaftimplantats) beispielsweise relativ zu einer Längsachse des Oberschenkelknochens angegeben. Wenn einmal eine geeignete Orientierung für die Gelenkpfanne bestimmt worden ist, kann basierend hierauf eine geeignete, dazu passende Orientierung des Schaftimplantats bestimmt werden, um den gewünschten Bewegungsbereich des Gelenks nach der Implantation zu gewährleisten. Vorzugsweise wird das Verfahren so gestaltet, dass nach Implantation eines Teils des künstlichen Gelenks also beispielsweise der Gelenkpfanne, die Orientierung des implantierten Teils überprüft wird und insbesondere gemessen wird. Basierend auf der gemessenen Orientierung kann dann eine neue Berechnung oder eine Aktualisierung der bisher vorgenommenen Berechnung durchgeführt werden, falls die gemessene Orientierung nicht mit der vorher erfindungsgemäß bestimmten Orientierung übereinstimmt, also etwas davon abweicht. Somit kann die Bestimmung der geeigneten Orientierung für das zweite implantierte Teil des künstlichen Gelenks an die tatsächliche Orientierung des zuerst implantierten Teils des künstlichen Gelenks angepasst werden. Somit kann beispielsweise erreicht werden, dass trotz der Abweichung der Bewegungsbereich des künstlichen Gelenks den Bewegungsbereich des anatomischen Gelenks umfasst. Insbesondere kann auch die Orientierung des Schaftimplantats zuerst bestimmt werden und dann in einem zweiten Schritt die Orientierung der Gelenkpfanne. Insbesondere kann die Orientierung des Schaftimplantats nach Implantation im Oberschenkel verifiziert werden und die verifizierte (d.h. gemessene) Orientierung kann dann als Grundlage zur Berechnung der geeigneten Lage für die Gelenkpfanne genommen werden. Auch kann das Schaftimplantat zuerst implantiert werden und seine Orientierung verifiziert werden und danach eine Implantation der Gelenkpfanne erfolgen.

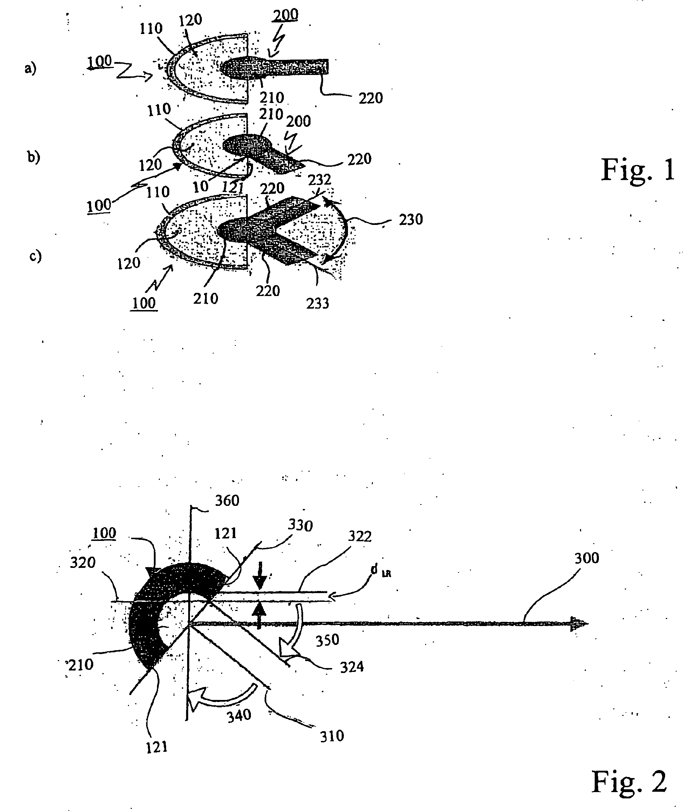

Hinsichtlich des oben als a) bezeichneten Analysekonzept wird noch Folgendes vorgebracht. Vorteilhafterweise berechnet eine Software die Maximalbereiche von Schlüsselbewegungen, z.B. die Bereiche von Flexions-/Extensionsbewegungen und Abduktions-/Adduktionsbewegungen. Diese Bereiche werden dann verwendet, um die bestmögliche Kombination einer Gelenkpfanne mit einem Gelenkkopf auszuwählen bzw. festzulegen. Verfügbare Kombinationen werden vorteilhafterweise von einer Datenbank bereitgestellt. Figur 1 zeigt ein Beispiel einer Kombination aus einem ersten und zweiten Teil eines künstlichen Gelenks 100, 200, die ein Maximalbereich einer Bewegung entlang eines Primärbogens 230 (so genannter "primary arc range") von 100° bereitstellt. Vorzugsweise werden die Gelenkpfannen 100 dergestalt implantiert, also in eine derartige Lage gebracht, dass der Maximalbereich ohne ein Anstoßen 10 erreicht werden kann. Ein "Anstoßen" 10 bedeutet hier, dass ein Teil 200 des künstlichen Gelenks im Rahmen einer Gelenkbewegung so anstößt, dass eine weitere Fortführung der Gelenkbewegung verhindert wird. Vorzugsweise wird also eine Ausgangslage oder Null-Lage des künstlichen Gelenks 100, 200 so platziert, dass diese in der Mitte des Maximalbereichs oder der Maximalbereiche des anatomischen Gelenks liegt. Die Figur 1a zeigt das künstliche Gelenk 100, 200 in einer derartigen Ausgangsposition. Die Figur 1b zeigt eine Situation, bei der der Rand eines Bewegungsbereichs erreicht ist, also ein Teil 200 des künstlichen Gelenks im Rahmen einer Gelenkbewegung an dem anderen Teil 100 (121) anstößt bzw. anliegt. Genauer stößt der Übergangsbereich zwischen Gelenkkugel 210 und Gelenkschaft 220 an der Endfläche 121 der Gelenkpfanne 100 bei dem Punkt 10 an. Die Gelenkpfanne 100 besteht aus einer äußeren Schale 110, in die ein zur Gelenkkugel passendes Futter 120 *(auch Liner genannt)* eingepasst ist. Figur 1c zeigt einen durch die Anstoßpunkte 10 definierten maximalen Bewegungsbereich 230 (primary arc range), der beispielsweise 100° betragen kann. Im Folgenden wird ein Beispiel vorgestellt:

Die maximale Flexion im Bereich zur Ausgangsposition beträgt beispielsweise 80°. Die maximale Extension verglichen zu der Ausgangssituation beträgt 15°. Dies führt zu einem maximalen Flexions-/Extensionsbereich von 95°. Da dieser Bereich von 95° kleiner ist, als der maximale Bewegungsbereich von 100° (primary arc range 100°) des künstlichen Gelenks der Figur 1, kann dieses künstliche Gelenk verwendet werden. Die Mitte dieses maximalen Flexions-/Extensionsbereichs beträgt demnach 47,5°. Diese Mittebeschreibt in einer Ebene die geeignete Orientierung für eine Platzierung der Gelenkpfanne hinsichtlich der Flexions-/Extensionsebene. Berechnet man diese geeignete Orientierung in Bezug auf die Ausgangssituation (Null-Situation), so würde dies einer Orientierung entsprechend, die zumindest 32,5° Flexion entspricht. Dies errechnet sich auch aus dem Mittelwert des maximalen Flexions-/Extensionsbereich von 47,5° minus dem maximalen Extensionsbereich von 15°.

Hinsichtlich des oben genannten Analysekriteriums b) wird noch Folgendes vorgebracht. Es wird davon ausgegangen, dass Daten gegeben sind, die die maximale Abduktion beschreiben. Beispielsweise ein Programm oder das erfindungsgemäße Verfahren verwendet diese Daten und berechnet darauf basierend die Richtung einer für diesen Fall möglichen Luxation. Dabei wird für diesen Fall angenommen, dass die Luxation entlang der Oberschenkelhalsachse des Femurs erfolgt. Die Oberschenkelhalsachse kann insbesondere aus gegebenen anatomischen Daten bestimmt werden, wie sie beispielsweise durch ein Computertomogramm (CT) ermittelt werden können. Somit ist die Lagebeziehung zwischen der maximalen Abduktionslage und der Oberschenkelhalsachse bekannt. Weiter kann vorzugsweise angenommen werden, dass für den Fall der Luxation kein Anstoßen zwischen dem ersten und zweiten Teil des künstlichen Gelenks, also in diesem Beispiel zwischen Kopf und Pfanne gegeben ist. Weiter sind vorzugsweise die geometrischen Daten, also insbesondere Daten über Form und/oder Gestalt des künstlichen Gelenks aus einer Datenbank bekannt. Weiter kann man annehmen, dass ein Teil des künstlichen Gelenks, z.B. eine Mittelachse der Gelenkpfanne und die Richtung der Luxation einen Winkel einschließen, der im folgenden Neigungswinkel genannt wird. Ein mechanischer Widerstand gegen die Luxation hängt von einem Abstand ab, der im folgenden Luxationskenngröße d_{LR} genannt wird. Dieser Widerstand hängt von dem Neigungswinkel ab. Dies ist näher in Figur 2 erläutert. In Figur 2 ist die Gelenkpfanne 100 gezeigt, in der eine halbkugelförmige Öffnung für den Kopf 210 eines Gelenkkopfes ist. Dieser Gelenkkopf 210 kann sich im Zustand der Verbindung mit der Gelenkpfanne 100 von der Gelenkpfanne 100 lösen. Dies wird als Luxation bezeichnet. Die Richtung der Luxation ist in Figur 2 mit 300 bezeichnet. Für diese Luxationsrichtung können verschiedene Richtungen angenommen werden. Vorzugsweise werden Richtungen angenommen, wie sie sich für Schlüssellagen des einen Teils des Gelenks relativ zu dem anderen Teil ergeben. Beispielsweise kann die Luxationsrichtung für den Zustand der maximalen Abduktion bestimmt werden. Insbesondere kann die Luxationsrichtung für diese Lage oder für andere Schlüssellagen bzw. Lagen so berechnet werden, dass angenommen wird, dass eine Luxation entlang der Oberschenkelhalsachse erfolgt. Somit kann die Luxationsrichtung identisch mit der Oberschenkelhalsachse für den Fall der maximalen Abduktion sein.

Vorzugsweise sind die geometrischen Daten für das künstliche Gelenk, insbesondere für ein ausgewähltes künstliches Gelenk aus einer Vielzahl von künstlichen Gelenken bekannt, also insbesondere in einer Datenbank gespeichert. Die Symmetrieachse, um die die Gelenkpfanne 100 der Figur 2 rotationssymmetrisch ist, ist in Figur 2 mit dem Bezugszeichen 310 bezeichnet. Diese Mittelachse 310 geht durch den Mittelpunkt der schalenförmigen Vertiefung der Gelenkpfanne 100 und steht insbesondere senkrecht auf einer Ebene 330, die die Endflächen 121 der Gelenkpfanne 100 verbindet. Die Mittelachse 310 entspricht der Orientierung des künstlichen Gelenks.

Eine so genannte Luxationsachse 320 ist parallel zur Richtung der Luxation 300. Sie ist weiterhin senkrecht zu einer Geraden 360, die senkrecht zur Richtung der Luxation ist und durch die Mediansagittalebene (vom Kopf zum Fuß) verläuft. Die angenommene Luxationsrichtung ist senkrecht dazu. Sie entspricht im Zustand der maximalen Abduktion der Oberschenkelhalsachse. Die Gerade 360 schneidet einen Schnittpunkt zwischen der Mittelachse 310 und der Ebene 330. Die Luxationsachse 320 schneidet weiterhin die Ebene 330 an einem Randpunkt der Endfläche 121 der Gelenkpfanne 100, und zwar den Randpunkt der Endfläche 121, der der Mittelachse 310 am nächsten ist. Der Winkel zwischen der Mittelachse 310 und der Geraden 360, die senkrecht zu der Luxationsachse 320 ist, ist der Neigungswinkel der Gelenkpfanne 100 und ist in Figur 2 mit 340 bezeichnet. Der Luxations-Neigungswinkel 350 ist ebenfalls in Figur 2 bezeichnet und ist der Winkel zwischen der Luxationsachse 320 und einer Geraden 324, die senkrecht zur Ebene 330 ist und die Ebene 330 an dem oben genannten Randpunkt der Endfläche 121 schneidet.

Der Überstand zwischen der Implantatkante und dem höchsten Punkt der Implantataushöhlung (in dem entsprechenden Fall) wird im Folgenden als Luxationskenngröße d_{LR} bezeichnet und führt zum mechanischen Widerstand, der der Luxation entgegengesetzt wird. Bei diesem Beispiel wird die Luxationskenngröße durch den Abstand d_{LR} angegeben, wobei der sich aus dem Abstand ergebende Widerstand um so größer ist je größer dieser Abstand ist. Der Abstand d_{LR} ist der Abstand zwischen der Luxationsachse 320 und einer dazu parallelen Geraden 322. Diese parallele Gerade 322 schneidet die Gerade 360, die senkrecht zur Luxationsachse 320 ist und die die Ebene 330 in der Mitte zwischen den Endflächen 121 schneidet.

In der Bestimmung des Wertes der benötigten Luxationskenngröße können neben geometrischen Eigenschaften des künstlichen Gelenks auch beispielsweise Materialeigenschaften des künstlichen Gelenks mit einbezogen werden, insbesondere Oberflächenreibungseigenschaften und Elastizität des Materials. Je elastischer bzw. flexibler das Material des künstlichen Gelenks ist, um so leichter kann eine Luxation bewirkt werden. Je geringer die Reibung zwischen dem einen Teil des künstlichen Gelenks und dem anderen Teil des künstlichen Gelenks also beispielsweise zwischen Gelenkkopf und Gelenkpfanne ist, umso leichter kann der eine Teil von dem anderen Teil getrennt werden.

Die Luxationskenngröße d_{LR} und, wie oben dargelegt, folglich der Neigungswinkel der Luxation wird erfindungsgemäß insbesondere dergestalt optimiert, dass eine maximale Abduktion ermöglicht wird, wobei es kein Anstoßen des einen Teil des Gelenks an den anderen gibt, und wobei weiter ein möglichst hoher Widerstand gegen eine Luxation gewährleistet ist.

Wenn einmal der optimale Widerstand, insbesondere der sich aus den geometrischen Eigenschaften ergebende Widerstand, gefunden wurde, so ist somit ein Luxations-Neigungswinkel 350 festgelegt, und aus diesem kann ein geeigneter Neigungswinkel 340 der künstlichen Gelenkpfanne und somit eine diesem Neigungswinkel entsprechende, geeignete Orientierung des künstlichen Gelenks bestimmt werden. In maximaler Abduktion wird die Luxationsrichtung festgelegt. Die Ausrichtung der Körperstrukturen Pelvis und Femur sind in der Position bekannt. Aus z.B. der Datenbank weiß man, welche minimale Luxationskenngröße d_{LR} man haben muss, um nicht zu luxieren. Diese minimalste Luxationskenngröße d_{LR} ergibt einen Neigungswinkel zwischen der Mittelachse 310 des künstlichen Gelenks und der Luxationsachse. Beispielsweise entspricht die Luxationsachse der Oberschenkelhalsachse oder die Beziehung zwischen der Luxationsachse und der Oberschenkelhalsachse ist bekannt (z.B. weil der Gelenkkopf bereits implantiert ist). Über die Oberschenkelhalsachse und den Neigungswinkel kann nun eine geeignete Lage der Mittelachse 310 und somit eine geeignete Orientierung des Gelenkpfannenimplantats ermittelt werden.

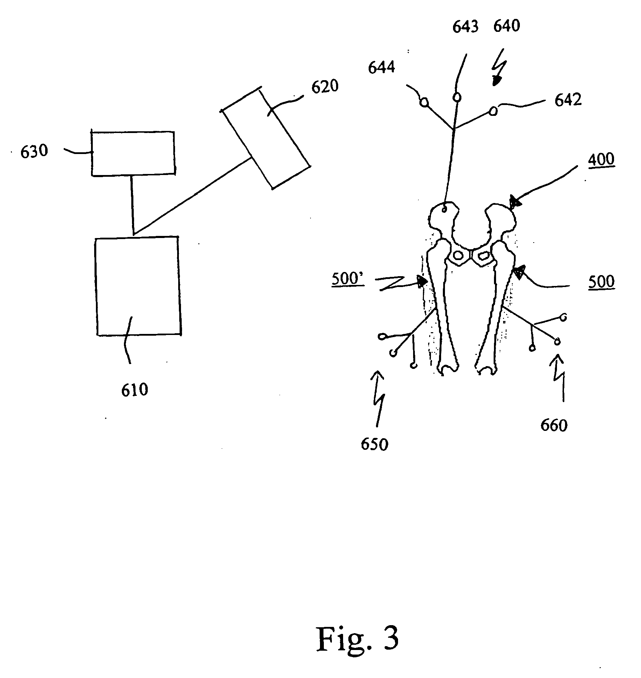

Figur 3 zeigt eine erfindungsgemäße Vorrichtung einer Datenverarbeitungseinrichtung 610. Sie ist mit einem Monitor 630 und einer Detektionseinrichtung 620 verbunden. Die Detektionseinrichtung 620 ist ausgebildet, Markereinrichtungen 640, 650 und 660 zu detektieren, die wie abgebildet, jeweils drei Kugeln aufweisen, die mit 642, 643 und 644 bezeichnet sind.

Die Markereinrichtungen sind am Becken und an jeweils einem Oberschenkelknochen angebracht. Wird der Oberschenkelknochen 500 bzw. 500' relativ zum Becken 400 bewegt, so können somit mögliche relative Lagen der Oberschenkelknochen 500 und 500' relativ zum Becken 400 erfasst werden. Insbesondere können typische Schlüssellagen und Extremlagen (Maximallagen) am Patienten, insbesondere während der Operation, erfasst werden, bevor eine Implantation durchgeführt wird.

Basierend auf den erfassten Daten kann dann ein künstliches Gelenk, beispielsweise eine Gelenkpfanne und eine Gelenkkopf implantiert werden. Insbesondere kann das künstliche Gelenk, insbesondere jeder Teil des künstlichen Gelenks ebenfalls mit einer Markereinrichtung versehen sein, um die exakte und gewünschte Platzierung zu gewährleisten. Die Vorrichtung kann ausgebildet sein, Hinweise für den Operateur zu geben, in welche Lage der Oberschenkelknochen 500 bzw. 500' relativ zu dem Becken 400 noch zu bringen ist, um ausreichend Datenmaterial zur Berechnung einer geeigneten Lage des künstlichen Gelenks zu haben. Insbesondere kann die Einrichtung 610, 620 und 630 auch dazu genutzt werden, das künstliche Gelenk nach Bestimmung der geeigneten Orientierung und insbesondere entsprechend dem Ergebnis des erfindungsgemäßen Planungsverfahrens zu platzieren, indem der Operateur durch Hinweise geleitet wird und/oder indem ein Implantationsroboter gesteuert wird.

## Patentansprüche

1. Verfahren zum Bestimmen einer Orientierung eines anatomischen Gelenks für die Planung einer Implantation von mindestens einem künstlichen Gelenk (100, 200) in einem menschlichen oder tierischen Körper, wobei ein erster Teil (100) des künstlichen Gelenks und ein zweiter Teil (200) des künstlichen Gelenks ausgebildet sind, miteinander eine künstliche Gelenkverbindung eingehen zu können, wobei der erste Teil (100) zur Implantation in einer ersten Körperstruktur (400) des menschlichen oder tierischen Körpers vorgesehen ist und der zweite Teil (200) zur Implantation in einer zweiten Körperstruktur (500, 500') des menschlichen oder tierischen Körpers vorgesehen ist, um ein anatomisches Gelenk (400, 500; 400, 500'), das die erste Körperstruktur (400) mit der zweiten Körperstruktur (500) verbindet oder verbunden hat, zu ersetzen, wobei das Verfahren den folgende Schritt umfasst:
- Körperstruktur-Daten werden bereitgestellt, die eine Bewegbarkeit der ersten Körperstruktur (400) relativ zur zweiten Körperstruktur (500)beschreiben, und durch den folgenden Schritt **gekennzeichnet** ist:
- mindestens ein Implantat-Datensatz wird bereitgestellt, der eine Bewegbarkeit des ersten Teils relativ zum zweiten Teils betrifft, und der Daten (d_{LR}),die die Hemmung oder den Widerstand gegen eine relative Bewegung der ersten und zweiten Körperstruktur betreffen, umfasst,
wobei basierend auf den Körperstruktur-Daten und dem mindestens einen Implantat-Datensatz, eine für die Implantation geeignete Orientierung des künstlichen Gelenks bestimmt wird.

2. Verfahren nach Anspruch 1, bei welchem basierend auf den Körperstrukturdaten eine Orientierung des künstlichen Gelenks bestimmt wird und basierend auf der bestimmten Orientierung des künstlichen Gelenks die für die Implantation des künstlichen Gelenks geeignete Orientierung bestimmt wird.

3. Verfahren nach Anspruch 1 oder 2, bei welchem der Implantat-Datensatz folgendes umfasst:
- mindestens einen möglichen Bewegungsbereich (230) des mindestens einen künstlichen Gelenks (100, 200); und/oder
- mögliche Lagen (350) des ersten Teils relativ zum zweiten Teil; und/oder
- Daten über die Geometrie des künstlichen Gelenks.

4. Verfahren nach einem der vorhergehenden Ansprüche, bei welchem die durch die Körperstruktur-Daten umfasste Bewegbarkeit folgendes umfasst:
- mindestens einen mit dem anatomischen Gelenk (400, 500, 500') möglichen Bewegungsbereich; und/oder
- mögliche Lagen der ersten Körperstruktur (400) relativ zur zweiten Körperstruktur (500, 500'); und/oder
- Daten über die Geometrie des anatomischen Gelenks; und/oder
- Daten über die Hemmung einer oder über einen Widerstand gegen eine relative Bewegung der ersten und zweiten Körperstruktur; und/oder
- Daten über mindestens eine Luxationsrichtung.

5. Verfahren nach Anspruch 4, bei welchem die Bestimmung der geeigneten Lage so ausgestaltet ist, dass, im Falle der Platzierung des künstlichen Gelenks (100, 200) mit der geeigneten Orientierung, der mögliche Bewegungsbereich des anatomischen Gelenks (400, 500, 500') durch den möglichen Bewegungsbereich des künstlichen Gelenks (100, 200) umfasst ist oder zumindest in etwa entspricht.

6. Verfahren nach einem der vorhergehenden Ansprüche, bei welchem die anatomischen Daten überprüft werden, indem sie mit vorgegebenen Daten verglichen werden, die anatomische übliche oder maximal mögliche Bewegbarkeiten und/oder mögliche relative Lagen der Körperstrukturen bei einem Menschen oder einem Tier darstellen.

7. Verfahren nach einem der vorhergehenden Ansprüche, bei welchem mehr als ein Implantat-Datensatz bereitgestellt wird, wobei jeder Implantat-Datensatz eines von verschiedenen künstlichen Gelenken (100, 200) beschreibt, und basierend auf den Körperstruktur-Daten und den Implantat-Datensätzen sowohl mindestens ein geeignetes künstliches Gelenk (100, 200) ausgewählt wird als auch die mindestens eine geeignete Orientierung für die Implantation des mindestens einen ausgewählten künstlichen Gelenks (100, 200) bestimmt wird.

8. Verfahren nach einem der vorhergehenden Ansprüche, bei welchem mittels eines Navigationssystems und/oder mittels einer Detektionseinrichtung (620) und/oder mittels medizinischer Analyse und/oder Diagnoseeinrichtungen die Körperstruktur-Daten zumindest teilweise bestimmt werden.

9. Verfahren nach einem der vorhergehenden Ansprüche, bei welchem zumindest eine Markereinrichtung (640) an der ersten und/oder zweiten Körperstruktur (400, 500, 500') angebracht wird und/oder angebracht ist und die Lage und/oder eine Bewegung der ersten und/oder zweiten Körperstruktur (400, 500, 500') durch Detektion der mindestens einen Markereinrichtung (640) erfasst wird, um die Körperstruktur-Daten zu bestimmen.

10. Planungsverfahren zum Planen einer Implantation eines zweiteiligen künstlichen Gelenks (100, 200) in einem menschlichen Körper, wobei das Planungsverfahren das Verfahren nach einem der vorhergehenden Ansprüche verwendet wird, um eine geeignete Orientierung für das Implantieren des künstlichen Gelenks (100, 200) zu bestimmen.

11. Computerprogramm, das, wenn es in einen Computer geladen wird, oder auf einen Computer läuft, den Computer veranlasst die Schritte des Verfahrens oder Planungsverfahrens nach einem der vorhergehenden Ansprüche auszuführen.

12. Computerlesbares Speichermedium, auf dem das Computerprogramm nach Anspruch 11 gespeichert ist oder physikalische Signalwelle, die als Information das Programm nach Anspruch 11 trägt.

13. Vorrichtung zum Bestimmen einer für eine Implantation geeigneten Orientierung von mindestens einem künstlichen Gelenk (100, 200) in einem menschlichen oder tierischen Körper, wobei ein erster Teil (100) des künstlichen Gelenks und ein zweiter Teil (200) des künstlichen Gelenks ausgebildet sind, miteinander eine künstliche Gelenkverbindung eingehen zu können, wobei der erste Teil zur Implantation in einer ersten Körperstruktur (400) des menschlichen oder tierischen Körpers vorgesehen ist und der zweite Teil (500, 500') zur Implantation in einer zweiten Körperstruktur des menschlichen oder tierischen Körpers vorgesehen ist, um ein anatomisches Gelenk (400, 500; 400, 500'), das die erste Körperstruktur (400) mit der zweiten Körperstruktur (500) verbindet oder verbunden hat, zu ersetzen, wobei die Vorrichtung die folgendes umfasst:
einen Speicher
- zum Speichern von Körperstruktur-Daten, die eine Bewegbarkeit der ersten (400) und zweiten Körperstruktur (500, 500') betreffen und die Lage der ersten und/oder zweiten Körperstruktur beschreiben;
**gekennzeichnet durch** eine Datenverarbeitungseinrichtung (610), die ausgebildet ist,
basierend auf den Körperstruktur-Daten und auf einem Implantat-Datensatz, der eine Bewegbarkeit des ersten Teils relativ zum zweiten Teil betrifft, eine für die Implantation des ersten und/oder zweiten Teils des künstlichen Gelenks geeignete Orientierung zu bestimmen, und der Implantat-Datensatz Daten (d_{LR}), die die Hemmung oder einen Widerstand gegen eine relative Bewegung des ersten und zweiten Teils betreffen, umfasst, und die Bestimmung der geeigneten Orientierung so ausgestaltet ist, dass ein Widerstand gegen eine Luxation des künstlichen Gelenks optimiert wird und/oder der Widerstand dazu benutzt wird die geeignete Orientierung zu bestimmen oder ein bereits als geeignet bestimmte Orientierung zu bewerten und/oder zu verbessern.

14. Vorrichtung nach Anspruch 13, bei welcher weiter eine Detektionseinrichtung (620) vorgesehen ist, um die Körperstruktur-Daten zumindest teilweise zu bestimmen
wobei die Detektionseinrichtung (620) ausgebildet ist, die Lage und/oder eine Bewegung der ersten und/oder zweiten Körperstruktur (400, 500, 500') durch Detektion von mindestens einer Markereinrichtung (640), die an der ersten und/oder zweiten Körperstruktur (400, 500, 500') angebracht ist, zu erfassen.

15. Vorrichtung nach Anspruch 14, die weiter eine Hinweiseinrichtung (630) umfasst, die ausgebildet ist, basierend auf den gespeicherten Lagen und/oder Bewegungen einen Bediener auf die Lagen und/oder Bewegungen der ersten und/oder zweiten Körperstruktur hinzuweisen, die noch zu detektieren sind, um zur Bestimmung der Orientierung des anatomischen Gelenks und/oder der geeigneten Orientierung des künstlichen Gelenks noch erforderliche Körperstruktur-Daten zu gewinnen.

## Claims

1. Method of determining an orientation of an anatomical joint for planning an implantation of at least one artificial joint (100, 200) in a human or animal body, whereby a first part (100) of the artificial joint and a second part (200) of the artificial joint are designed to form an artificial joint connection with one another, and the first part (100) is intended to be implanted in a first body structure (400) of the human or animal body and the second part (200) is intended to be implanted in a second body structure (500, 500') of the human or animal body in order to replace an anatomical joint (400, 500; 400, 500') which connects or has connected the first body structure (400) to the second body structure (500), which method comprises the following step:
- preparing body structure data describing an ability of the first body structure (400) to move relative to the second body structure (500), and **characterised by** the following step:
- at least one implant data set relating to an ability of the first part to move relative to the second part is prepared and contains data (d_{LR}) relating to the inhibition of or resistance to a relative movement of the first and second body structure,
and an appropriate orientation of the artificial joint is determined for the implantation on the basis of the body structure data and the at least one implant data set.

2. Method as claimed in claim 1, whereby an orientation of the artificial joint is determined on the basis of the body structure data, and the appropriate orientation for implanting the artificial joint is determined on the basis of the determined orientation.

3. Method as claimed in claim 1 or 2, whereby the implant data set contains the following:
- at least one possible range of movement (230) of the at least one artificial joint (100, 200); and/or
- possible positions (350) of the first part relative to the second part; and/or
- data pertaining to the geometry of the artificial joint.

4. Method as claimed in one of the preceding claims, whereby the ability to move described by the body structure data comprises:
- at least one possible range of movement by means of the anatomical joint (400, 500, 500'); and/or
- possible positions of the first body structure (400) relative to the second body structure (500, 500'); and/or
- data pertaining to the geometry of the anatomical joint; and/or
- data pertaining to the inhibition of or a resistance to a relative movement of the first and second body structure: and/or
- data pertaining to at least one luxation direction.

5. Method as claimed in claim 4, whereby the appropriate position is determined in such a way that if the artificial joint (100, 200) is positioned on the basis of the appropriate orientation, the possible range of movement of the anatomical joint (400, 500, 500') is described by the possible range of movement of the artificial joint (100, 200) or at least approximately corresponds to it.

6. Method as claimed in one of the preceding claims, whereby the anatomical data is checked by comparing it with predefined data representing the usual or maximum possible anatomical movements and/or possible relative positions of the body structures in a human or animal.

7. Method as claimed in one of the preceding claims, whereby more than one implant data set is prepared, and each implant data set describes one of different artificial joints (100, 200), and both at least one appropriate artificial joint (100, 200) is selected and the at least one appropriate orientation for implanting the at least one selected artificial joint (100, 200) is determined on the basis of the body structure data and the implant data sets.

8. Method as claimed in one of the preceding claims, whereby at least some of the body structure data is determined by means of a navigation system and/or by means of a detection system (620) and/or by means of medical analysis and/or diagnostic systems.

9. Method as claimed in one of the preceding claims, whereby at least one marker device (640) is fitted and/or mounted on the first and/or second body structure (400, 500, 500') and the position and/or a movement of the first and/or second body structure (400, 500, 500') is detected by detecting the at least one marker device (640) in order to determine the body structure data.

10. Planning method for planning an implantation of a two-part artificial joint (100, 200) in a human body, which planning method uses the method as defined in one of the preceding claims as a means of determining an appropriate orientation for implanting the artificial joint (100, 200).

11. Computer programme which, when loaded onto a computer or run on a computer, prompts the computer to run the steps of the method or planning method as claimed in one of the preceding claims.

12. Computer-readable storage medium on which the computer programme as claimed in claim 11 is stored or physical signal wave which carries information in the form of the programme as claimed in claim 11.

13. Device for determining an appropriate orientation of at least one artificial joint (100, 200) for an implantation in a human or animal body, and a first part (100) of the artificial joint and a second part (200) of the artificial joint are designed to form an artificial joint connection with one another, and the first part is intended to be implanted in a first body structure (400) of the human or animal body and the second part (500, 500') is intended to be implanted in a second body structure of the human or animal body with a view to replacing an anatomical joint (400, 500; 400, 500') which connects or has connected the first body structure (400) to the second body structure (500), which device comprises the following:
a memory
- for storing body structure data relating to an ability of the first (400) and second body structure (500, 500') to move and describing the position of the first and/or second body structure;
**characterised by** a data processing system (610) which is programmed to determine an appropriate orientation for implanting the first and/or second part of the artificial joint on the basis of the body structure data and an implant data set relating to the ability of the first part to move relative to the second part, and the implant data set contains data (d_{LR}) relating to the inhibition of or a resistance to a relative movement of the first and second part, and the appropriate orientation is determined so that a resistance to a luxation of the artificial joint is optimised and/or the resistance is used as a means of determining the appropriate orientation or evaluating and/or improving an orientation which has already been determined as being appropriate.

14. Device as claimed in claim 13 in which a detection system (620) is also provided as a means of determining at least some of the body structure data,
and the detection system (620) is designed to detect the position and/or a movement of the first and/or second body structure (400, 500, 500') by detecting at least one marker device (640) mounted on the first and/or second body structure (400, 500, 500').

15. Device as claimed in claim 14 which also has an indicator system (630) which is programmed to indicate to a user the positions and/or movements of the first and/or second body structure which still need to be detected, based on the stored positions and/or movements of the first and/or second body structure, in order to obtain the body structure data still needed to determine the orientation of the anatomical joint and/or the appropriate orientation of the artificial joint.

## Revendications

1. Procédé pour déterminer l'orientation d'une articulation anatomique pour programmer l'implantation d'au moins une articulation artificielle (100, 200) dans un corps humain ou animal, dans lequel une première partie (100) de l'articulation individuelle et une seconde partie (200) de l'articulation individuelle sont conçues de manière à pouvoir former ensemble une liaison artificielle d'articulation, dans lequel la première partie (100) est prévue pour être implantée dans une première structure corporelle (400) du corps humain ou animal, et la seconde partie (200) est prévue pour être implantée dans une seconde structure corporelle (500, 500') du corps humain ou animal, afin de remplacer une articulation anatomique (400, 500 ; 400', 500') qui relie ou a relié la première structure corporelle (400) à la seconde structure corporelle (500), le procédé comprenant l'étape suivante :
- préparer des données de structure corporelle qui décrivent une mobilité de la première structure corporelle (400) par rapport à la seconde structure corporelle (500), et étant **caractérisé par** l'étape suivante :
- préparer au moins un ensemble de données d'implant qui concerne une mobilité de la première partie par rapport à la seconde partie et qui comprend des données (d_{LR}) qui concernent le blocage ou la résistance à l'égard d'un mouvement relatif de la première et de la seconde structure corporelle, procédé dans lequel, sur la base des données de structure corporelle et du au moins un ensemble de données d'implant, on détermine une orientation de l'articulation artificielle, qui convient à l'implantation.

2. Procédé selon la revendication 1, dans lequel, sur la base des données de structure corporelle, on détermine une orientation de l'articulation artificielle et, sur la base de l'orientation déterminée de l'articulation artificielle, on détermine l'orientation qui convient à l'implantation de l'articulation artificielle.

3. Procédé selon l'une quelconque des revendications 1 ou 2, dans lequel l'ensemble de données d'implant comprend ce qui suit :
- au moins une plage de mouvement possible (230) de la au moins une articulation artificielle (100, 200) ; et/ou
- des positions possibles (350) de la première partie par rapport à la seconde partie ; et/ou
- des données relatives à la géométrie de l'articulation artificielle.

4. Procédé selon l'une quelconque des revendications 1 à 3, dans lequel la mobilité décrite par les données de structure corporelle, comprend ce qui suit :
- au moins une plage de mouvement possible avec l'articulation anatomique (400, 500, 500') ; et/ou
- des positions possibles de la première structure corporelle (400) par rapport à la seconde structure corporelle (500, 500') ; et/ou
- des données relatives à la géométrie de l'articulation anatomique ; et/ou
- des données relatives au blocage ou à une résistance à l'égard d'un mouvement relatif de la première et de la seconde structure corporelle ; et/ou
- des données relatives à une direction de luxation.

5. Procédé selon la revendication 4, dans lequel la détermination de la position qui convient est conçue de manière que, lorsque l'articulation artificielle (100, 200) est placée avec l'orientation qui convient, la plage de mouvement possible de l'articulation anatomique (400, 500, 500') est comprise par la plage de mouvement possible de l'articulation artificielle (100, 200) ou correspond au moins à celle-ci.

6. Procédé selon l'une quelconque des revendications 1 à 5, dans lequel on vérifie les données anatomiques en les comparant à des données prédéfinies qui représentent des mobilités anatomiques usuelles ou au maximum possibles et/ou des positions relatives possibles des structures corporelles chez un homme ou un animal.

7. Procédé selon l'une quelconque des revendications 1 à 6, dans lequel on prépare plus d'un ensemble de données d'implant, chaque ensemble de données d'implant décrivant l'une de différentes articulations artificielles (100, 200), et, sur la base des données de structure corporelle et des ensembles de données d'implant, on sélectionne au moins une articulation artificielle (100, 200) qui convient et on détermine aussi la au moins une orientation qui convient pour l'implantation de la au moins une articulation artificielle (100, 200) sélectionnée.

8. Procédé selon l'une quelconque des revendications 1 à 7, dans lequel on détermine au moins en partie les données de structure corporelle, au moyen d'un système de navigation et/ou au moyen d'un dispositif de détection (620) et/ou au moyen d'une analyse médicale et/ou de dispositifs de diagnostic.

9. Procédé selon l'une quelconque des revendications 1 à 8, dans lequel on place au moins un dispositif de repérage (640) sur la première et/ou la seconde structure corporelle (400, 500, 500'), et/ou celui-ci est placé, et on relève la position et/ou un mouvement de la première et/ou de la seconde structure corporelle (400, 500, 500') par détection du au moins un dispositif de repérage (614), afin de déterminer les données de structure corporelle.

10. Procédé de programmation pour programmer l'implantation d'une articulation artificielle (100, 200) en deux parties dans un corps humain, le procédé de programmation utilisant le procédé selon l'une quelconque des revendications 1 à 9, pour déterminer une orientation convenable pour l'implantation de l'articulation artificielle (100, 200).

11. Programme d'ordinateur qui, lorsqu'il est chargé dans un ordinateur ou tourne sur un ordinateur, fait exécuter à l'ordinateur les étapes du procédé ou du procédé de programmation selon l'une quelconque des revendications 1 à 10.

12. Support de mémoire lisible par un ordinateur sur lequel est mémorisé le programme d'ordinateur selon la revendication 11 ou onde de signaux physiques qui porte le programme selon la revendication 11 comme information.

13. Dispositif pour déterminer une orientation convenant à l'implantation d'au moins une articulation artificielle (100, 200) dans un corps humain ou animal, dans lequel une première partie (100) de l'articulation individuelle et une seconde partie (200) de l'articulation individuelle sont conçues de manière à pouvoir former ensemble une liaison artificielle d'articulation, dans lequel la première partie (100) est prévue pour être implantée dans une première structure corporelle (400) du corps humain ou animal, et la seconde partie (200) est prévue pour être implantée dans une seconde structure corporelle (500, 500') du corps humain ou animal, afin de remplacer une articulation anatomique (400, 500 ; 400', 500') qui relie ou a relié la première structure corporelle (400) à la seconde structure corporelle (500), le dispositif comprenant ce qui suit :
une mémoire pour mémoriser des données de structure corporelle qui concernent une mobilité de la première structure corporelle (400) et de la seconde structure corporelle (500, 500') et qui décrivent la position de la première et/ou de la seconde structure corporelle ;
**caractérisé par** un dispositif de traitement de données (610) qui est conçu pour déterminer, sur la base des données de structure corporelle et d'un ensemble de données d'implant, qui concerne une mobilité de la première partie par rapport à la seconde partie, une orientation qui convient pour l'implantation de la première et/ou de la seconde partie de l'articulation artificielle, et l'ensemble de données d'implant comprend des données (d_{LR}) qui concernent le blocage ou une résistance à l'égard d'un mouvement relatif de la première et de la seconde partie, et la détermination de l'orientation qui convient est telle qu'une résistance à l'égard d'une luxation de l'articulation artificielle est optimisée et/ou la résistance est utilisée pour déterminer l'orientation qui convient, ou pour évaluer et/ou améliorer une orientation déjà déterminée comme convenable.

14. Dispositif selon la revendication 13, dans lequel il est prévu en outre un dispositif de détection (620) pour déterminer au moins en partie les données de structure corporelle, dans lequel le dispositif de détection (620) est conçu pour relever la position et/ou un mouvement de la première et/ou de la seconde structure corporelle (400, 500, 500') par détection d'au moins un dispositif de repérage (640) qui est placé sur la première et/ou la seconde structure corporelle (400, 500, 500').

15. Dispositif selon la revendication 14 qui comprend en outre un dispositif de signalisation (630) qui est conçu pour signaler à un opérateur, sur la base des positions et/ou mouvements mémorisés, les positions et/ou mouvements de la première et/ou seconde structure corporelle qui doivent encore être détectés, afin d'obtenir encore des données de structure corporelle nécessaires pour déterminer l'orientation de l'articulation anatomique et/ou de l'orientation convenable de l'articulation artificielle.
